(19) Europäisches Patentamt
European Patent Office
Office européen des brevets

(11) Veröffentlichungsnummer: **0 279 218 B1**

# (12) EUROPÄISCHE PATENTSCHRIFT

(45) Veröffentlichungstag der Patentschrift: **19.12.90**

(51) Int. Cl.[5]: **A01N 43/64**

(21) Anmeldenummer: **88101004.5**

(22) Anmeldetag: **25.01.88**

(54) **Herstellung von Mitteln gegen Fischparasiten.**

(30) Priorität: **03.02.87 DE 3703103**

(43) Veröffentlichungstag der Anmeldung: **24.08.88 Patentblatt 88/34**

(45) Bekanntmachung des Hinweises auf die Patenterteilung: **19.12.90 Patentblatt 90/51**

(84) Benannte Vertragsstaaten: **DE FR GB IT**

(56) Entgegenhaltungen:
**DE-A- 2 313 721**
**DE-A- 2 650 014**
**DE-B- 2 413 722**

(73) Patentinhaber: **BAYER AG, D-5090 Leverkusen 1 Bayerwerk(DE)**

(72) Erfinder: **Mehlhorn, Heinz, Prof. Dr., Steinstrasse 12a, D-4040 Neuss-Üdesheim(DE)**
Erfinder: **Schmahl, Günter, Dr., Markstrasse 329, D-4630 Bochum(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 279 218 B1

**Beschreibung**

Die vorliegende Erfindung betrifft die Verwendung von Triazintrionen zur Herstellung von Mitteln gegen parasitäre Protozoen und Metazoen bei Fischen.

Zu den Protozoen und Metazoen gehören Klassen, die als Parasiten bei Fischen weit verbreitet sind. Bei der Massentierhaltung in großen Zuchtanlagen stellen sie ein ernstes Problem dar, da sich ein Befall rasch über den ganzen Bestand ausbreiten kann.

Einige parasitäre Protozoen und Metazoen heften sich auf Haut und Kiemen der Fische und verursachen dadurch Verletzungen der Haut durch die die Fische anfällig für Infektionen werdehn. Sie sind außerdem Vektoren für Virusinfektionen. Andere parasitäre Protozoen und Metazoen befallen innere Organe der Fische z.B. Darm, Knochen und führen zu Verwachsungen oder zum Tod der Fische.

Mittel zur Bekämpfung der parasitären Protozoen und Metazoen sind bekannt, wirken jedoch nicht immer voll befriedigend. Außerdem besitzen sie meist mur ein enges Wirkungsspektrum.

Es wurde gefunden, daß die bekannten Triazintrione der Formel (I)

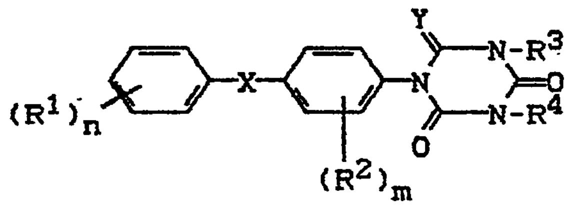

(I)

in welcher
X für O oder S steht,
Y für O oder S steht,
$R^1$ für gleiche oder verschiedene Reste aus der Gruppe Halogen, Nitro, CN, Amino, Alkyl, Halogenalkyl, Alkoxy, Halogenalkoxy, Alkylthio, Halogenalkylthio, Alkylsulfonyl, Alkylsulfinyl, Halogenalkylsulfonyl, Halogenalkylsulfinyl, Acyl, Carboxy, Carbonylamino, Carbonylalkoxy, Carbamoyl, Sulfamoyl steht,
$R^2$ für gleiche oder vrschiedene Reste aus der bei $R^1$ angegebenen Gruppe von Substituenten,
n und m für ganze Zahlen von 0 bis 3 stehen,
$R^3$ für Wasserstoff oder Alkyl steht,
$R^4$ für Wasserstoff oder Alkyl steht,
zur Herstellung von Mitteln gegen Ektoparasiten bei Fischen verwendet werden können.

Die Verbindungen der Formel (I) sind bekannt oder können nach an sich bekannten Methoden hergestellt werden (DE-OS 2 413 722; DE-OS 2 313 721, DE-OS 2 718 799, US-P 4 219 552). Es ist bekannt, daß diese Verbindungen gegen Coccidien wirksam sind. Es ist jedoch nichts über ihre Verwendung gegen Protozoen und Metazoen bei Fischen bekannt.

Bevorzugt sind Verbindungen der Formel (I) in welcher
X und Y für O stehen,
$R^1$ und $R^2$ unabhängig voneinander für gleiche oder verschiedene Reste aus der Gruppe Halogen, insbesondere Fluor, Chlor, Brom, Nitro, CN, Amino, $C_{1-4}$-Alkyl, insbesondere Methyl oder Ethyl, $C_{1-4}$-Halogenalkyl, insbesondere Trifluormethyl, Trichlormethyl, Fluor-chlorethyl, $C_{1-4}$-Alkoxy, insbesondere Methylendioxy, Isopropoxy, Methoxy, $C_{1-4}$-Halogenalkoxy, insbesondere Trifluormethoxy, Difluormethylendioxy, Tetrafluorethylendioxy, $C_{1-4}$-Alkylthio, insbesondere Methylenmercapto, Ethylenmercapto, $C_{1-4}$-Halogenalkylthio, insbesondere Trifluormethylthio, $C_{1-4}$-Alkylsulfonyl, insbesondere Methylsulfonyl, $C_{1-4}$-Alkylsulfinyl, $C_{1-4}$-Halogenalkylsulfonyl und -sulfinyl, insbesondere Trifluormethylsulfonyl, $C_{1-4}$-Acyl insbesondere Acetyl, Propionyl, Benzoyl, $C_{1-4}$-Carbonylalkoxy insbesondere Methoxycarbonyl, Ethoxycarbonyl, stehen,
n und m für ganze Zahlen von 0 bis 2 stehen,
$R^3$ und $R^4$ unabhängig voneinander für Wasserstoff oder $C_{1-4}$-Alkyl insbesondere Methyl stehen.

Besonders bevorzugt werden Verbindungen der Formel I eingesetzt,
in welcher
$R^4$ für Wasserstoff steht,
$R^3$ für $C_{1-4}$-Alkyl steht,
Y für Sauerstoff oder Schwefel steht,
X für Sauerstoff steht,
$R^2$ für gleiche oder verschiedene Reste aus der Gruppe $C_{1-4}$-Alkyl, Halogen wie insbesondere Chlor oder Brom, $C_{1-4}$-Alkoxy und $C_{1-2}$-Halogenalkyl steht,
$R^1$ für gleiche oder verschiedene Reste aus der Gruppe Halogen insbesondere Clor, Brom, $NO_2$, $C_{1-4}$-Alkyl, $C_{1-2}$-Halogenalkyl, $C_{1-4}$-Alkylmercapto, das gegebenenfalls durch Halogen substituiert ist, $C_{1-4}$-Alkoxy, das gegebenenfalls durch Halogen substituiert ist, $C_{1-4}$-Alkylsulfinyl, das gegebenenfalls

durch Halogen substituiert ist, $C_{1-4}$-Alkylsulfonyl, das gegebenenfalls durch Halogen substituiert ist steht,
n und m für ganze Zahlen von 0-2 stehen.

Besonders bevorzugt sind Verbindungen der Formel I,
in welcher
$R^4$ für Wasserstoff steht,
$R^3$ für Methyl steht,
X und Y für Sauerstoff stehen,
$R^2$ für gleiche oder verschidene Reste aus der Gruppe Chlor, Brom, Methyl, Trifluormethyl, Methoxy, Ethoxy steht,
$R^1$ für insbesondere in 4-Stellung gleiche oder verschiedene Reste aus der Gruppe Trifluormethylmercapto, Trifluormethylsulfinyl, Trifluormethylsulfonyl, Trifluormethoxy, steht,
n und m unabhängig voneinander für 0 oder 1 stehen.

Folgende Verbindungen seien beispielhaft genannt, ohne die Erfindung in irgendeiner Weise einzuschränken:
1-[3,5-Dichlor-4-(4'-trifluormethoxy-phenoxy)-phenyl]-3-methyl-1,3,5-triazin-2,4,6(1H, 3H, 5H)-trion,
1-[3,5-Dichlor-4-(4'-trifluormethylthio-phenoxy)-phenyl]-3-methyl-1,3,5-triazin-2,4,6(1H, 3H, 5H)-trion,
1-[4-(4'-Trifluormethylthio-phenoxy)-phenyl]-3-methyl-1,3,5-triazin-2,4,6(1H, 3H, 5H)-trion,
1-[3-Chlor-5-brom-4-(4'-triflormethylthio-phenoxy)-phenyl]-3-methyl-1,3,5-triazin-2,4,6(1H,3H, 5H)-trion,
1-[3,5-Dibrom-4(4'-trifluormethylthio-phenoxy)-phenyl]-3-methyl-1,3,5-triazin-2,4,6(1H, 3H, 5H)-trion,
1-[3,5-Dichlor-4-(3'-methyl-4'-trifluormethylthiophenoxy)-phenyl]-3-methyl-1,3,5-triazin-2,4,6(1H, 3H, 5H)-trion,
1-[4-(4'-trifluormethylsulfonyl-phenoxy)-phenyl]-3-methyl-1,3,5-triazin-2,4,6(1H, 3H, 5H)-trion,
1-[3,5,-Dichlor-4-(4'-trifluormethylsulfonyl-phenoxy)-phenyl]-3-methyl-1,3,5-triazin-2,4,6(1H, 3H, 5H)-trion,
1-[3-Chlor-5-brom-4-(4'-trifluormethylsulfonyl-phenoxy)-phenyl]-3-methyl-1,3,5-triazin-2,4,6(1H, 3H, 5H)-trion,
1-[3,5-Dichlor-4-(2'-Chlor-4'-trifluormethylsulfonyl-phenoxy)-phenyl]-3-methyl-1,3,5-triazin-2,4,6(1H, 3H, 5H)-trion,
1-[3-Methoxy-4-(4'-trifluormethylthio-phenoxy)-phenyl]-3-methyl-1,3,5-triazin-2,4,6-(1H, 3H, 5H)-trion,
1-[4-(4'-Trifluormethylsulfinyl-phenoxy)-phenyl]-3-methyl-1,3,5-triazin-2,4,6(1H, 3H, 5H)-trion,
1-[3,5-Dichlor-4-(4'-trifluormethylsulfinyl-phenoxy)-phenyl]-3-methyl-1,3,5-triazin-2,4,6(1H, 3H, 5H)-trion,
1-[4-(3'-Trifluormethylsulfonyl-phenoxy)-3,5-dimethylphenyl]-3-methyl-1,3,5,-triazin-2,4,6(1H, 3H, 5H)-trion,
1-[3-Chlor-4-(4'-trifluormethylsulfonyl-phenoxy)-phenyl]-3-methyl-1,3,5-triazin-2,4,6(1H, 3H, 5H)-trion,
1-[3-Methyl-4-(4'-trifluormethylthio-phenoxy)-phenyl]-3-methyl-1,3,5-triazin-2,4,6-(1H, 3H, 5H)-trion,
1-[3,5-Dichlor-4-(2'-methyl-4'-trifluormethylsulfonylphenoxy)-phenyl]-3-methyl-1,3,5-triazin-2,4,6(1H, 3H, 5H)-trion,
1-[3-Chlor-5-methyl-4-(2'-chlor-4'-trifluormethylsulfonyl-phenoxy)-phenyl]-3-methyl-1,3,5-triazin-2,4,6(1H, 3H, 5H)-trion,
1-[4-(4'-Trifluormethylsulfonyl-phenoxy)-3,5-dimethylphenyl]-3-methyl-1,3,5-triazin-2,4,6(1H, 3H, 5H)-trion,
1-[3-Chlor-5-methyl-4-(4'-trifluormethylsulfonylphenoxy)-phenyl]-3-methyl-1,3,5-triazin-2,4,6(1H, 3H,-5H)-trion,
1-[3-Chlor-4-(2'-Chlor-4'-trifluormethylthio-phenoxy)-phenyl]-3-methyl-1,3,5,-triazin-2,4,6(1H, 3H, 5H)-trion,
1-[3-Brom-4-(4'-trifluormethylsulfonyl-phenoxy)-phenyl]-3-methyl-1,3,5-triazin-2,4,6(1H, 3H, 5H)-trion,
1-[3-Chlor-5-trifluormethyl-4-(4'-trifluormethylsulfonyl-phenoxy)-phenyl]-3-methyl-1,3,5-triazin-2,4,6(1H, 3H, 5H)-trion,
1-[3,5-Dichlor-4(4'-trifluormethylsulfonyl-phenoxy)-phenyl]-3-methyl-2-thioxo-4,6-dioxo-1,3,5(1H, 3H, 5H)-triazin,
1-[3-Methyl-4-(4'-trifluormethylthio-phenoxy)-phenyl]-3-methyl-2-thioxo-4,6-dioxo-1,3,5-(1H, 3H, 5H) triazin,
1-[3-Methyl-4-(6-trifluormethyl-benzthiazol-2-yloxy)-phenyl]-3-methyl-1,3,5-triaxin-2,4,6-(1H, 3H, 5H)-trion.

Ganz besonders hervorgehoben sei Toltrazuril common name für 1-[3-Methyl-4-(4'-trifluormethylthio-phenoxy)-phenyl]-3-methyl-1,3,5-triazin-2,4,6(1H, 3H, 5H)-trion.

Es war bekannt, daß sich die Triazintrione der Formel (I) zur Bekämpfung von Coccidien der Säugetiere und des Geflügels einsetzen lassen. Es war nichts darüber bekannt, daß sie auch eingesetzt werden können um Parasiten bei Fischen zu bekämpfen.

Zu den Parasiten bei Fischen gehören aus den Stamm der Protozoen Spezies der Klasse Ciliata z.B. Ichthyophthirius multifiliis, Chilodonella cypini, Trichodina spp., Glossatella spp., Epistylis spp. der Klasse Myxosporidia z.B. Myxosoma cerebralis, Myxidium, Myxobolus, Hemeguya, Hoterellus, der Klasse der Mikrosporidia z.B. Glugea spp., Thelohania spp., Pleistophora spp., aus dem Stamm der Plathelminthen Monogenea z.B. Dactylogyrus spp., Gyrodactylus spp., Pseudodactylogyrus spp., Diplozoon spp.

Zu den Fischen gehören Nutz-, Zucht-, Aquarien- und Zierfische aller Altersstufen die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus caphalus), Pompano, Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut z.B. Karpfen von 2-4 cm Körperlänge. Sehr gut geeignet sind die Mittel auch in der Aalmast.

Die Behandlung der Fische erfolgt entweder oral z.B. über das Futter oder durch Kurzzeitbehandlung, "medizinisches Bad" in das die Fische eingesetzt und in dem sie eine zeitlang (Minuten bis mehrere Stunden) z.B. beim Umsetzen von einem Zuchtbecken zum anderen gehalten werden.

Es kann aber auch eine vorübergehende oder dauernde Behandlung des Lebensraums der Fische z.B. ganzer Teichanlagen, Aquarien, Tanks oder Becken in denen die Fische gehalten werden erfolgen.

Der Wirkstoff wird in Zubereitungen verarbreicht, die den Anwendungen angepaßt sind.

Zubereitungen zur oralen Anwendungen sind Pulver, Granulate, Lösungen, Emulsions- oder Suspensionskonzentrate die als Futterzusätze mit dem Futter homogen vermischt werden.

Die Zubereitungen werden in an sich bekannter Weise herstellt indem man den Wirkstoff mit festen oder flüssigen Trägerstoffen gegebenenfalls unter Zusatz weiterer Wirkstoffe wie Emulgier- oder Dispergiermittel, Lösungsvermittler, Farbstoffe, Antoxidantien, Konservierungsstoffe vermischt.

Zu den festen Trägerstoffen zählen z.B. natürliche Gesteinsmehle wie Kaoline, Tonerden, Talkum, Kreide, Diatomeenerde, organische Trägerstoffe wie Zucker, Rohr-, Milch-, Traubenzucker, Getreideprodukte wie Getreidemehle oder -schrote, Stärke, Tiermehle, Cellulose, Milchpulver, anorganische Trägerstoffe wie Kochsalz, Carbonate wie Calciumcarbonat, Hydrogencarbonate, Aluminiumoxide, Kieselsäure, Silikate.

Zu den flüssigen Trägerstoffen und Lösungsvermittlern zählen:
Wasser, Alkanole wie Ethanol, Isopropanol, Glykole wie Ethylenglykol, Propylenglykol, Polyethylenglykole, Polypropylenglykole und ihre Copolymere, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmonobutylether, Ketone wie Aceton, Methylethylketon, aromatische und/oder aliphatische Kohlenwasserstoffe, pflanzliche oder synthetische Öle, DMF, DMSO, Dimethylacetamid, N-Methylpyrrolidon, 2-Dimethyl-4-oxymethylen-1,3-dioxalon.

Zu den Dispergier- und Emulgiermitteln zählen:
nichtionogene Tenside wie polyoxyethyliertes Rizinusöl, polyoxyethyliertes Sorbitan-monoleat, Sorbitanmonostearat, Glycerinmonostearat, Polyoxyethylenstearat, Alkylphenolpolyglykolether, ampholytische Tenside wie Di-Na-N-lauryl-β-iminodipropionat oder Lecithin, anionactive Tenside wie Na-Laurylsulfat, Fettalkoholethersulfate, Mono/Dialkylpolyglykoletherorthophosphorsäureester-monoethanolaminsalze, kationaktive Tenside wie Cetyltrimethylammoniumchlorid.

Die Konzentration des Wirkstoffs, liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teilchbehandlung) der Fische sind Lösungen des Wirkstoffs in einem oder mehreren polaren Lösungsmitteln, die bei Verdünnen mit Wasser alkalisch reagieren.

Zur Herstellung der Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel suspendiert sein und sich erst im Wasser lösen. Dabei soll das Wasser nach Zusatz der Wirkstofflösung einen pH-Wert von 7-10, vorzugsweise aber einen pH-Wert von 8-10 haben.

Die Konzentration des Wirkstoffes kann im Bereich von 0,5-50 % liegen, vorzugsweise aber in einem Bereich von 1-25%.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykol, Poly(oxoethylen)-poly(oxypropylen)-Polymere, basische Alkohole wie Mono-, Di- und Triethanolamin, Ketone wie Aceton oder Methylethylketon, Ester wie Milchsäureethylester ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D,L-Arginin, L- bzw. D, L-Lysin, Methylglucosamin, Glucosamin, 2-Amino-2-hydroxymethylpropandiol-(1,3) ferner wie N,N,N',N'-tetrakis-(2-hydroxypropyl)-ethylendiamin oder Polyether-Tetrol auf der Basis Ethylendiamin (M.G. 480-420), anorganische Basen, wie Ammoniak oder Natriumcarbonat-gegebenenfalls unter Zugabe von Wasser.

Die Zubereitungen können auch 0,1 bis 20 Gew.-%, vorzugsweise 0,1-10 Gew.-% anderer Formulierhilfsstoffe, wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2-50 mg Wirkstoff pro Liter Wasser bevorzugt 5-10 mg pro Liter, bei einer Behandlungsdauer von 3-4 Stunden. Bei der Behandlung von jungen Karpfen wird z.B. mit einer Konzentration von 5-10 mg/l und einer Behandlungsdauer von ca. 1-4 Stunden gearbeitet. Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1-5 mg Wirkstoff pro Liter Wasser verwendet werden.

Zubereitungen zur Anwendung als Futterzusatz sind z.B. wie folgt zusammengesetzt:

| | |
|---|---|
| a) Wirkstoff der Formel I | 1 - 10 Gewichtsteile |
| Sojabohnen-Protein | 49 - 90 Gewichtsteile |
| b) Wirkstoff der Formel I | 0,5 - 10 Gewichtsteile |
| Benzylalkohol | 0,08 - 1,4 Gewichtsteile |
| Hydrozypropyl methylcellulose | 0 - 3,5 Gewichtsteile |
| Wasser Rest ad 100 | |

Zubereitungen zur Anwendung bei "medizinischen Bädern" und zur Teichbehandlung sind z.B. wie folgt zusammengesetzt und hergestellt.

c) 2,5 g Toltrazuril werden in 100 ml Triethanolamin unter Erwärmen gelöst.

d) 2,5 g Toltrazuril und
12,5 g Milchsäure werden in 100 ml Triethanolamin unter Erwärmen und Rühren gelöst.

e) 10,0 g Toltrazuril wird in 100 ml Monoethanolamin gelöst.

| | |
|---|---|
| f) Toltrazuril | 5,0 g |
| Propylenglykol | 50,0 g |
| Natriumcarbonat | 5,0 g |
| Wasser | ad 100 ml |
| g) Toltrazuril | 5,0 g |
| Monoethanolamin | 10 g |
| N-Methylpyrrolidon | ad 100 ml |
| h) Toltrazuril | 2,5 g |
| Natriumcarbonat | 5,0 g |
| Polyethylenglykol 200 | ad 100 ml |

Toltrazuril wird unter Erwärmen im Polyethylenglykol gelöst und Natriumcarbonat darin suspendiert.

Beispiel 1

In-vivo Fischbehandlung

Fische, die stark mit Ektoparasiten infiziert waren, wurden für 1 Stunden bei 20°C in 20 l Wasser das mit 10 ppm Toltrazuril versetzt war behandelt. Danach wurden die Fische getötet und ihre Parasitenbefall überprüft. Es ergab sich folgendes Bild:

| Anzahl | Fischspezies | Parasitenspezies | Beobachtung am Parasit nach 1 h |
|---|---|---|---|
| 9 | Rotaugen | Dactylogyrus cornu | Parasiten am Boden des Behälters schwer geschädigt oder tot, vereinzelte geschädigte Parasiten auf den Kiemen |
| 15 | Karpfen (2–4 cm) | Dactylogyrus vastator | Parasiten am Boden des Behälters tot, geschädigte Parasiten in den Kiemen |
| 4 | Aale (44–48 cm) | Pseudodactylogyrus anguillae | Behandlungsdauer 2,5 h; Parasiten geschädigt |

In-vitro Parasitenbehandlung

Parasiten der angegebenen Spezies wurden in einer Glasschale in 150 ml Wasser von 20°C gegeben, das mit der angegebenen Konzentration Toltrazuril versetzt war. Nach der angegebenen Zeit wurden die Parasiten unter dem Lichtmikroskop überprüft. Dabei wurden folgende Beobachtungen gemacht:

| Parasit | Konz./Zeit | Beobachtung am Parasit |
|---|---|---|
| Diplozoon-paradoxum | 5 ppm/55 min. | tot |
| " | 10ppm/4 min. | tot |
| Myxosoma sp. | 20 ppm/4,5 h | Parasit letal geschädigt |

**Patentansprüche**

Verwendung von Triazintrionen der Formel I

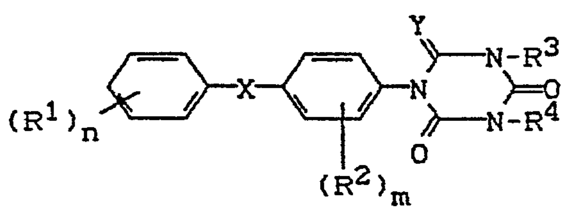

(I)

in welcher
X für O oder S steht,
Y für O oder S steht,
$R^1$ für gleiche oder verschiedene Reste aus der Gruppe Halogen, Nitro, CN, Amino, Alkyl, Halogenalkyl, Alkoxy, Halogenalkoxy, Alkylthio, Halogenalkylthio, Alkylsulfonyl, Alkylsulfinyl, Halogenalkylsulfonyl, Halogenalkylsulfinyl, Acyl, Carboxy, Carbonylamino, Carbonylalkoxy, Carbamoyl, Sulfamoyl steht,
$R^2$ für gleiche oder verschiedene Reste aus der bei $R^1$ angegebenen Gruppe von Substituenten,
n und m für ganze Zahlen von 0 bis 3 stehen,
$R^3$ für Wasserstoff oder Alkyl steht,
$R^4$ für Wasserstoff oder Alkyl steht,
zur Herstellung von Mitteln gegen Ektoparasiten bei Fischen.

## Claims

Use of triazinetriones of the formula I

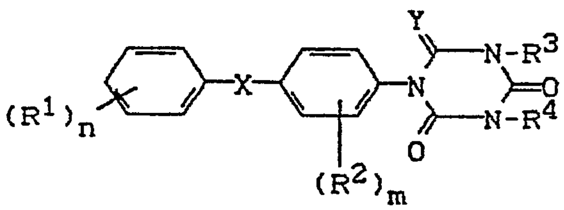

(I)

in which
X represents O or S,
Y represents O or S,
$R^1$ represents identical or different radicals from the group comprising halogen, nitro, CN, amino, alkyl, halogenoalkyl, alkoxy, halogenoalkoxy, alkylthio, halogenoalkylthio, alkylsulphonyl, alkylsulphinyl, halogenoalkylsulphonyl, halogenoalkylsulphinyl, acyl, carboxyl, carbonylamino, carbonylalkoxy, carbamoyl and sulphamoyl,
$R^2$ represents identical or different radicals from the group of substituents mentioned for $R^1$,
n and m represent integers from 0 to 3,
$R^3$ represents hydrogen or alkyl and
$R^4$ represents hydrogen or alkyl,
for the preparation of agents against ectoparasites in fish.

## Revendications

Utilisation de triazine-triones de formule I

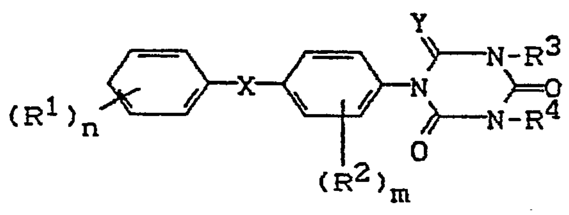

(I)

dans laquelle
X représente O ou S,
Y représente O ou S,
$R^1$ représente des restes, identiques ou différents, du groupe halogéno, nitro, CN, amino, alkyle, halogénalkyle, alkoxy, halogénalkoxy, alkylthio, halogénalkylthio, alkylsulfonyle, alkylsulfinyle, halogén alkylsulfonyle, halogénalkylsulfinyle, acyle, carboxy, carbonylamino, carbonylalkoxy, carbamoyle, sulfamoyle,
$R^2$ représente des restes, identiques ou différents, du groupe de substituants indiqué pour $R^1$,
n et m sont des nombres entiers de 0 à 3,
$R^3$ représente l'hydrogène ou un groupe alkyle,
$R^4$ représente l'hydrogène ou un groupe alkyle,
pour la préparation de compositions contre des ectoparasites de poissons.